# EUROPEAN PATENT SPECIFICATION

(11) **EP 0 668 872 B1**
(45) Date of publication and mention of the grant of the patent: **13.05.1998**
(21) Application number: 93922734.4
(22) Date of filing: 28.09.1993
(51) Int. Cl.: C07K 14/435, A61K 38/17

(54) **CALCIUM CHANNEL BLOCKING POLYPEPTIDES FROM THERAPHOSIDAE APHONOPELMA**
KALZIUM-KANAL-BLOCKIERENDE POLYPEPTIDE AUS THERAPHOSIDAE APHONOPELMA
POLYPEPTIDES PROVENANT DU VENIN DE LA THERAPHOSIDAE APHONOPELMA ET BLOQUANT LES CANAUX CALCIQUES

(30) Priority: 03.11.1992 US 973323
(43) Date of publication of application: 30.08.1995
(73) Proprietor: PFIZER INC., New York, N.Y. 10017 (US); NPS PHARMACEUTICALS, INC., Salt Lake City Utah 84108 (US)
(72) Inventor: NASON, Deane, M., II, Norwich, CT 06360 (US); PHILLIPS, Douglas, Gales Ferry, CT 06335 (US); SACCOMANO, Nicholas, A., Ledyard, CT 06339 (US); VOLKMANN, Robert, A., Mystic, CT 06355 (US)
(74) Representative: Hayles, James Richard
(86) International application number: US9309069
(87) International publication number: WO9410196

(56) References cited:
- BIOLOGICAL CHEMISTRY HOPPE-SEYLER vol. 370, no. 5, May 1989, Berlin, DE, pp. 484-498, A. SAVEL-NIEMANN
- CHEMICAL ABSTRACTS, vol. 68, no. 23, 3 June 1968, Columbus, Ohio, US, abstract no. 101945w, H.L. STAHNKE et al.

### SEQUENCE LISTING

### (1) GENERAL INFORMATION:

(i) APPLICANT:
   (A) NAME: Pfizer Inc
   (B) STREET: 235 East 42nd Street
   (C) CITY: New York
   (D) STATE: New York
   (E) COUNTRY: U.S.A.
   (F) POSTAL CODE (ZIP): 10017
   (G) TELEPHONE: (203) 441-4905
   (H) TELEFAX: (203) 441-5221

   (A) NAME: NPS Pharmaceuticals, Inc.
   (B) STREET: 420 Chipeta Way
   (C) CITY: Salt Lake City
   (D) STATE: Utah
   (E) COUNTRY: U.S.A.
   (F) POSTAL CODE (ZIP): 84108
   (G) TELEPHONE: (801) 583-4939
   (H) TELEFAX: (801) 583-4961
(ii) TITLE OF INVENTION: CALCIUM CHANNEL BLOCKING POLYPEPTIDES FROM THERAPHOSIDAE APHONOPELMA
(iii) NUMBER OF SEQUENCES: 7
(iv) COMPUTER READABLE FORM:
   (A) MEDIUM TYPE: Floppy disk
   (B) COMPUTER: IBM PC compatible
   (C) OPERATING SYSTEM: PC-DOS/MS-DOS
   (D) SOFTWARE: PatentIn Release #1.0, Version #1.25 (EPO)
(vi) PRIOR APPLICATION DATA:
   (A) APPLICATION NUMBER: US 07/973,323
   (B) FILING DATE: 03-NOVEMBER-1992

### (2) INFORMATION FOR SEQ ID NO:1:

(i) SEQUENCE CHARACTERISTICS:
   (A) LENGTH: 39 amino acids
   (B) TYPE: amino acid
   (C) STRANDEDNESS: single
   (D) TOPOLOGY: linear
(ii) MOLECULE TYPE: peptide
(iii) HYPOTHETICAL: NO
(iv) ANTI-SENSE: NO
(vi) ORIGINAL SOURCE:
   (A) ORGANISM: Theraphosidae aphonopelma
   (F) TISSUE TYPE: venom
(xi) SEQUENCE DESCRIPTION: SEQ ID NO:1:
   Leu Phe Glu Cys Val Leu Ser Cys Asp Ile Lys Lys Asn Gly Lys Pro
   1               5                   10                  15
   Cys Lys Pro Lys Gly Glu Lys Lys Cys Ser Gly Gly Trp Arg Cys Lys
               20                  25                  30
   Ile Asn Phe Cys Leu Lys Val
           35

### (2) INFORMATION FOR SEQ ID NO:2:

(i) SEQUENCE CHARACTERISTICS:
   (A) LENGTH: 39 amino acids
   (B) TYPE: amino acid
   (C) STRANDEDNESS: single
   (D) TOPOLOGY: linear
(ii) MOLECULE TYPE: peptide
(iii) HYPOTHETICAL: NO
(iv) ANTI-SENSE: NO
(vi) ORIGINAL SOURCE:
   (A) ORGANISM: Theraphosidae aphonopelma
   (F) TISSUE TYPE: venom
(xi) SEQUENCE DESCRIPTION: SEQ ID NO:2:
   Leu Phe Glu Cys Ala Leu Ser Cys Asp Ile Lys Lys Asn Gly Lys Pro
   1               5                   10                  15
   Cys Lys Pro Lys Gly Glu Lys Lys Cys Ser Gly Gly Trp Arg Cys Lys
               20                  25                  30
   Ile Asn Phe Cys Leu Lys Ile
           35

### (2) INFORMATION FOR SEQ ID NO:3:

(i) SEQUENCE CHARACTERISTICS:
   (A) LENGTH: 33 amino acids
   (B) TYPE: amino acid
   (C) STRANDEDNESS: single
   (D) TOPOLOGY: linear
(ii) MOLECULE TYPE: peptide
(iii) HYPOTHETICAL: NO
(iv) ANTI-SENSE: NO
(vi) ORIGINAL SOURCE:
   (A) ORGANISM: Theraphosidae aphonopelma
   (F) TISSUE TYPE: venom
(xi) SEQUENCE DESCRIPTION: SEQ ID NO:3:
   Cys Ala Glu Phe Gln Ser Lys Cys Lys Lys Asp Ser Glu Cys Cys Gly
   1               5                   10                  15
   Thr Leu Glu Cys Ser Pro Thr Trp Lys Trp Cys Val Tyr Pro Ser Pro
               20                  25                  30
   Phe

### (2) INFORMATION FOR SEQ ID NO:4:

(i) SEQUENCE CHARACTERISTICS:
   (A) LENGTH: 34 amino acids
   (B) TYPE: amino acid
   (C) STRANDEDNESS: single
   (D) TOPOLOGY: linear
(ii) MOLECULE TYPE: peptide
(iii) HYPOTHETICAL: NO
(iv) ANTI-SENSE: NO
(vi) ORIGINAL SOURCE:
   (A) ORGANISM: Theraphosidae aphonopelma
   (F) TISSUE TYPE: venom
(xi) SEQUENCE DESCRIPTION: SEQ ID NO:4:
   Ser Cys Gly His Val Gly Thr Pro Cys Glu Lys Asn Trp Asp Cys Cys
   1               5                   10                  15
   Lys Gly Lys Val Cys Ser Pro Arg Trp Lys Leu Cys Ala Tyr Glu Ser
               20                  25                  30
   Pro Phe

### (2) INFORMATION FOR SEQ ID NO:5:

(i) SEQUENCE CHARACTERISTICS:
   (A) LENGTH: 42 amino acids
   (B) TYPE: amino acid
   (C) STRANDEDNESS: single
   (D) TOPOLOGY: linear
(ii) MOLECULE TYPE: peptide
(iii) HYPOTHETICAL: NO
(iv) ANTI-SENSE: NO
(vi) ORIGINAL SOURCE:
   (A) ORGANISM: Theraphosidae aphonopelma
   (F) TISSUE TYPE: venom
(xi) SEQUENCE DESCRIPTION: SEQ ID NO:5:
   Cys Leu Gly Glu Asn Val Pro Cys Asp Lys Asp Arg Pro Asn Cys Cys
   1               5                   10                  15
   Ser Lys Tyr Glu Cys Leu Glu Pro Thr Gly Tyr Gly Arg Cys Tyr Ala
               20                  25                  30
   Ser Tyr Tyr Ser Tyr Lys Lys Lys Thr Leu
           35                  40

### (2) INFORMATION FOR SEQ ID NO:6:

(i) SEQUENCE CHARACTERISTICS:
   (A) LENGTH: 39 amino acids
   (B) TYPE: amino acid
   (C) STRANDEDNESS: single
   (D) TOPOLOGY: linear
(ii) MOLECULE TYPE: peptide
(iii) HYPOTHETICAL: NO
(iv) ANTI-SENSE: NO
(vi) ORIGINAL SOURCE:
   (A) ORGANISM: Theraphosidae aphonopelma
   (F) TISSUE TYPE: venom
(xi) SEQUENCE DESCRIPTION: SEQ ID NO:6:
   Leu Ile Glu Cys Ala Phe Ser Cys Asp Ile Thr Lys Asn Gly Lys Pro
   1               5                   10                  15
   Cys Lys Pro Lys Gly Glu Lys Lys Cys Ser Gly Gly Trp Arg Cys Lys
               20                  25                  30
   Ile Asn Phe Cys Leu Lys Ile
           35

### (2) INFORMATION FOR SEQ ID NO:7:

(i) SEQUENCE CHARACTERISTICS:
   (A) LENGTH: 35 amino acids
   (B) TYPE: amino acid
   (C) STRANDEDNESS: single
   (D) TOPOLOGY: linear
(ii) MOLECULE TYPE: peptide
(iii) HYPOTHETICAL: NO
(iv) ANTI-SENSE: NO
(vi) ORIGINAL SOURCE:
   (A) ORGANISM: Theraphosidae aphonopelma
   (F) TISSUE TYPE: venom
(xi) SEQUENCE DESCRIPTION: SEQ ID NO:7:
   Leu Phe Glu Cys Ala Leu Ser Cys Asp Ile Lys Lys Asn Gly Lys Pro
   1               5                   10                  15
   Cys Lys Pro Xaa Gly Glu Lys Lys Cys Ser Gly Gly Xaa Arg Xaa Xaa
               20                  25                  30
   Ile Asn Phe
           35

## Description

### Background of the Invention

This invention relates to polypeptides found in the venom of the Theraphosidae aphonopelma spider. The polypeptides and the pharmaceutically acceptable salts thereof block calcium channels in cells including neuronal and muscle cells of various organisms including invertebrates and vertebrates. This invention also relates to the use of said polypeptides and their salts in blocking calcium channels in cells such as cells in the nervous and muscular system of an organism, per se, and in the treatment of calcium channel mediated diseases and conditions in a mammal. Further, this invention relates to compositions comprising said polypeptides and salts thereof.

Compounds which are calcium antagonists have a variety of utilities. Calcium antagonists can find clinical application in the treatment of such conditions as angina, hypertension, cardiomyopathies, supraventricular arrhythmias, aesophogeal achalasia, premature labor and Raynaud's disease among others. See W. G. Nayler, Calcium Antagonists, Academic Press, Harcourt Brace Jovanovich Publishers, New York, NY 1988, the teachings of which are -incorporated herein by reference. Further, such compounds are useful in the study of the physiology of cells such as neuronal and muscle cells.

A polypeptide, isolated from tarantula venom, of the same general nature as SEQ ID NO:1 is disclosed in Biol. Chem. Hoppe-Seyler, vol. 370(5), 484-98 (1989). This polypeptide exhibits 79.5% homology (31/39 amino acids) with SEQ ID NO:1.

### Summary of the Invention

This invention concerns polypeptides found in the venom of the Theraphosidae aphonopelma spider. The polypeptides of this invention and the fractions in which they are present according to this invention are as follows.

Aphonopelma peptide 6-6 has the amino acid sequence, SEQ ID NO:1.
Aphonopelma peptide 6-8 has the amino acid sequence, SEQ ID NO:2.
Aphonopelma peptide 7-6.1 has the amino acid sequence, SEQ ID NO:3.
Aphonopelma peptide 7-13.1 has the amino acid sequence, SEQ ID NO:4.
Aphonopelma peptide 7-13.2 has the amino acid sequence, SEQ ID NO:5.
Aphonopelma peptide 7-15.2 has the amino acid sequence, SEQ ID NO:6.
Aphonopelma peptide 7-17.3 has the amino acid sequence, SEQ ID NO:7.

The polypeptides of this invention block calcium channels in cells. Accordingly, these polypeptides are useful in blocking calcium channels in cells, per se. These polypeptides are also useful in the control of invertebrate pests and in the treatment of diseases and conditions in a mammal mediated by calcium channel function in cells.

This invention also concerns pharmaceutical compositions comprising said polypeptides and methods of administering said polypeptides.

### Detailed Description of the Invention

Venom is obtained from the Theraphosidae aphonopelma spider through the process of milking by electrical stimulation according to standard methods well known to those skilled in the art. It is preferred that the method employed is one which safeguards against contamination of the whole venom by abdominal regurgitant or hemolymph. Such methods are well known to those skilled in the art. The whole venom so obtained is stored in a frozen state at about -78°C until used for purification as described below. Purification of the constituents from the whole venom is accomplished by reverse phase high performance liquid chromatography (HPLC) on a variety of preparative and semi-preparative columns such as C-4 and C-18 Vydac® columns (Rainin Instrument Co. Inc., Mack Road, Woburn Massachusetts 01801). Peak detection is carried out monochromatically at 220-230 nm. Further analysis of the fractions can be accomplished with, for example, polychrome UV data collected with a Waters 990 diode array detector (Millipore Corporation, Waters Chromatography Division, 34 Maple Street, Milford, Massachusetts 01757). The fractions from the columns are collected by known methods such as through the use of an ISCO/"FOXY" fraction collector and an ISCO 2159 peak detector (ISCO, 4700 Superior, Lincoln, Nebraska 68504). The fractions are collected in appropriately sized vessels such as sterile polyethylene laboratoryware. Concentration of the fractions is then accomplished by lyophilization from the eluant followed by lyophilization from water. Purity of the resulting constituent fractions then can be determined by chromatographic analysis using an analytical column with a gradient system which is more isocratic than the system used in the final purification of the fractions.

The polypeptides of the invention can be sequenced according to known methods. A general strategy for determining the primary structure includes, for example, the following steps. 1) Reduction and S-pyridylation of disulfide-bridged cysteine residues to enhance substrate susceptibility to enzymatic attack. 2) Controlled cleavage of the peptide through single or multi-step enzymatic digestion. 3) Isolation and purification of peptide fragments via reverse phase high performance liquid chromatography (HPLC). 4) Characterization of peptide fragments through N-terminal sequencing and ion-spray mass spectrometry.

S-pyridylethylation of cysteine residues of the polypeptides under study can be performed, for example, in solution followed by amino acid sequencing of the polypeptides. One such procedure for S-pyridylethylation can be accomplished as described below.

About 1 to 10 µg of polypeptide is dissolved or diluted in up to 50 µl of a buffer prepared by mixing 1 part 1 M TrisHCl, pH 8.5, containing 4 mM EDTA and 3 parts 8M guanidine·HCl. 2.5 µl of 10% aqueous 2-mercaptoethanol is added and the mixture is incubated at room temperature in the dark under argon for two hours. After incubation, 2 µl of 4-vinylpyridine (fresh reagent stored under argon at -20°C) is added and the mixture is incubated for another two hours at room temperature in the dark under argon. The mixture is then desalted, preferably by chromatography on a short, reverse phase column. The recovered alkylated polypeptide is then sequenced according to known methods.

Given the benefit of the disclosure herein with respect to the peptides present in fractions 6-6, 6-8, 7-6.1, 7-13.1, 7-13.2, 7-15.2, and 7-17.3 of venom from Theraphosidae aphonopelma, it is now possible to obtain said peptides by methods other than through isolation/purification from whole venom. The polypeptides of this invention can be produced using recombinant DNA techniques through the cloning of a coding sequence for said polypeptides or portions thereof. For example, hybridization probes which take advantage of the now known amino acid sequence information of said polypeptides can be employed according to methods well known to those skilled in the art to clone a coding sequence for the entire polypeptide. A combination of recombinant DNA techniques and in vitro protein synthesis can also be employed to produce the polypeptides of this invention. Such in vitro protein synthesis methods include, but are not limited to, use of an ABI 430A solid phase peptide synthesizer (Applied Biosystems, Inc., 850 Lincoln Center Drive, Foster City, California 94404) employing standard Merrifield chemistry or other solid phase chemistries well known to those skilled in the art.

The polypeptides of this invention block calcium channels present in a variety of cells such as cells in the nervous and muscular system of invertebrates and vertebrates.

The ability of the polypeptides of this invention to block calcium channels is demonstrated by the following procedure. Cerebellar granule cells are prepared from the cerebellum of 8 day old rats (Wilkin et al., Brain Res, **115**, 181-199, 1976). Squares (1 cm²) of Aclar (Proplastics Inc., 5033 Industrial Ave., Wall, NJ 07719) are coated with poly-L-lysine and placed in 12-well dishes that contain 1 ml of Eagles Basal Medium. The cells are dissociated and aliquots containing 6.25 x 10⁶ cells are added to each well containing the squares of Aclar. Cytosine-beta-D-arabino furanoside (final concentration 10 µM) is added 24 hours after plating. The cells are used for fura2 analysis at 6, 7 and 8 days of culture. The cells (attached to the Aclar squares) are transferred to 12 well dishes containing 1 ml of 2 µM fura2/AM (Molecular Probes Inc., Eugene, OR 97402) in HEPES buffer (containing 0.01% bovine serum albumin, 0.01% dextrose, pH 7.4, magnesium-free). The cells are incubated for 40 minutes at 37°C; the fura2/AM-containing buffer is removed and replaced with 1 ml of the same buffer without fura2/AM. To a quartz cuvette is added 2.0 ml of prewarmed (37°C) buffer. The cells on the Aclar are placed in the cuvette and the cuvette is inserted in a thermostatted (37°C) holder equipped with a magnetic stirrer and the fluorescence is measured with a fluorescence spectrophotometer (Biomedical Instrument Group, University of Pennsylvania). The fluorescence signal is allowed to stabilize for about two minutes. Then 5-20 µl of a stock solution of the compound under study in phosphate buffered saline (PBS, pH 7.4) at appropriate concentration is added to the cuvette. Calibration of the fluorescent signals and fura2/AM leakage correction are performed using the established procedures of Nemeth et al., J. Biol. Chem., **262**, 5188 (1987) at the completion of each test. The maximum fluorescence value (Fmax) is determined by addition of ionomycin (35 µM) and the minimum fluorescence value (Fmin) is determined by the subsequent addition of EGTA (12 mM) to chelate calcium. Employing the foregoing procedure, calcium channel blocking by a subject polypeptide is shown to occur by a decrease in fluorescence upon addition of the subject polypeptide. The polypeptides of the invention exhibit low IC₅₀ values, under 200 nm, for blocking calcium channels using this assay. For comparison, two known commercial calcium channel antagonists, Nifedipine and Verapamil, have IC₅₀ values of 33 nm and 4800 nm, respectively.

The polypeptides of this invention are useful as calcium channel blockers in cells, per se. As such, these polypeptides are also useful in the control of invertebrate pests and in the treatment of diseases and conditions mediated by calcium channels function in cells in a mammal such as angina, hypertension, cardiomyopathies, supraventricular arrhythmias, aesophogeal achalasia, premature labor and Raynaud's disease. Further, these polypeptides are useful in the study of the physiology of cells including, but not limited to, cells of the nervous, muscular and cardiovascular system.

Also within the scope of this invention are the pharmaceutically acceptable salts of the polypeptides of this invention. Such salts are formed by methods well known to those skilled in the art. For example, acid salts of the polypeptides can be prepared according to conventional methods.

When a polypeptide of this invention is to be administered to a mammal, it can be administered alone or in combination with pharmaceutically acceptable carriers or diluents in a pharmaceutical composition according to standard pharmaceutical practice. The polypeptides can be administered orally or parenterally with the parenteral route of administration being preferred for polypeptides. Parenteral administration includes intravenous, intramuscular, intraperitoneal, subcutaneous and topical administration.

For oral use of a polypeptide of this invention, the compound can be administered, for example, in the form of tablets or capsules, or as an aqueous solution or suspension. In the case of tablets for oral use, carriers which are commonly used include lactose and corn starch, and lubricating agents, such as magnesium stearate, are commonly added. For oral administration in capsule form, useful diluents are lactose and dried corn starch. When aqueous suspensions are required for oral use, the active ingredient is combined with emulsifying and suspending agents. If desired, certain sweetening and/or flavoring agents can be added.

For intramuscular, intraperitoneal, subcutaneous and intravenous use, sterile solutions of the active ingredient are usually prepared, and the pH of the solutions should be suitably adjusted and buffered. For intravenous use, the total concentration of solutes should be controlled to render the preparation isotonic.

When a polypeptide or salt thereof of this invention is used in a human subject, the daily dosage will normally be determined by the prescribing physician. Moreover, the dosage will vary according to the age, weight and response of the individual patient, as well as the severity of the patient's symptoms and the potency of the particular compound being administered.

When a polypeptide or salt thereof of this invention is used in control of invertebrate pests, said polypeptide is administered to said invertebrate directly or provided to the environment of said invertebrate. For example, a compound of this invention can be sprayed as a solution onto said invertebrate. The amount of compound necessary for control of said invertebrate will vary according to the invertebrate and environmental conditions and will be determined by the person applying the compound.

When a polypeptide or salt thereof of this invention is used in the physiological study of cells, said polypeptide is administered to the cells according to methods well known to those skilled in the art. For example, said polypeptide can be administered to cells in an appropriate physiological buffer. An appropriate concentration of a polypeptide of this invention for use in such studies is 200 µM. However, the concentration of said polypeptide in such studies may be greater than or much less than 200 µM. The amount of the polypeptide administered will be determined by the person skilled in the art according to well known methods.

### Examples

### Step I, Fractionation of Crude Aphonopelma Venom

Crude Theraphosidae aphonopelma venom (~40 µl) was applied to a reversed phase HPLC column (Vydac®, C-18, 300 Å, 22 x 250 mm) and was operated using a biphasic linear gradient program from 95% A and 5% B to 80% A and 20% B over 30 minutes, then from 80% A and 20% B to 30% A and 70% B over 25 minutes (A = 0.1% trifluoroacetic acid (TFA), B = acetonitrile) with detection at 220 nm and a flow rate of 15 ml/minute. Fractions were collected as set forth below.

**TABLE 1**

| | |
|---|---|
| Fraction 6 | 35.9 to 37.3 minutes. |
| Fraction 7 | 37.3 to 39.5 minutes. |
| Fraction 8 | 39.5 to 40.1 minutes. |
| Fraction 9 | 40.1 to 40.9 minutes. |
| Fraction 10 | 40.9 to 41.8 minutes. |

Each of the above fractions, from pooled runs, was concentrated by lyophilization. Further fractionations were performed as described below.

### Step II, Subfractionation of Step I Fractions

### A) Subfractionation of Fraction 6

The Fraction 6 material from Step 1, above, derived from ~60 µl of crude venom, was applied to a reversed phase HPLC column (Baker WP C-18, 4.6 x 250 mm) and was operated using a linear gradient program from 85% A and 15% B to 65% A and 35% B over 45 minutes (A = 0.1% TFA, B = acetonitrile) with detection at 220 nm and a flow rate of 1.0 ml/minute. Fractions were collected as set forth below.

**TABLE 2**

| | |
|---|---|
| Fraction 6-6 | 24.0 to 24.8 minutes. |
| Fraction 6-7 | 25.0 to 25.4 minutes. |
| Fraction 6-8 | 25.6 to 26.5 minutes. |
| Fraction 6-9 | 27.0 to 28.7 minutes. |
| Fraction 6-13 | 33.5 to 34.3 minutes. |
| Fraction 6-14 | 34.4 to 35.4 minutes. |

Each of the above fractions, from pooled runs, was concentrated by lyophilization.

### B) Subfractionation of Fraction 7

The Fraction 7 material from Step 1, above, derived from ~60 µl of crude venom, was applied to a strong cation exchange column (Sulfoethyl aspartamide (The Nest Group, 45 Valley Rd., Southborough, MA 01772), 5 µ, 4.6 x 200 mm) and was operated using a linear gradient program from 100% A and 0% B to 0% A and 100% B over 45 minutes (A = 5 mM H₃PO₄/20% acetonitrile, B = 5 mM H₃PO₄, 1.0 M NaCl/20% acetonitrile) with detection at 230 nm and a flow rate of 1.0 ml/minute. Fractions were collected as set forth below.

**TABLE 3**

| | |
|---|---|
| Fraction 7-6 | 21.9 to 22.3 minutes. |
| Fraction 7-9 | 24.8 to 25.5 minutes. |
| Fraction 7-11 | 26.1 to 26.7 minutes. |
| Fraction 7-12 | 27.1 to 27.6 minutes. |
| Fraction 7-13 | 27.6 to 28.9 minutes. |
| Fraction 7-14 | 29.7 to 30.8 minutes. |
| Fraction 7-15 | 31.0 to 32.3 minutes. |
| Fraction 7-16 | 32.5 to 33.1 minutes. |
| Fraction 7-17 | 34.2 to 36.0 minutes. |

Each of the above fractions, from pooled runs, was concentrated by lyophilization.

### C) Subfractionation of Fraction 8

The Fraction 8 material from Step 1, above, derived from ~100 µl of crude venom, was applied to a reversed phase HPLC column (Vydac®, C-18, 300 Å, 10 x 250 mm) and was operated using a linear gradient program from 80% A and 20% B to 71% A and 29% B over 35 minutes, then 71% A and 29% B for 10 minutes (A = 0.1% TFA, B = acetonitrile) with detection at 220 nm and a flow rate of 6.0 ml/minute. Fractions were collected as set forth below.

**TABLE 4**

| | |
|---|---|
| Fraction 8-5 | 19.2 to 21.2 minutes. |
| Fraction 8-6 | 21.3 to 22.5 minutes. |
| Fraction 8-7 | 25.9 to 26.5 minutes. |
| Fraction 8-8 | 27.4 to 28.3 minutes. |

Each of the above fractions, from pooled runs, was concentrated by lyophilization.

### Step III, Subfractionation of Step II fractions

### A) Subfractionation of Fraction 7-6

The Fraction 7-6 material from Step II-B, above, derived from ~70 µl of crude venom, was applied to a reversed phase HPLC column (Vydac®, C-18, 300 Å, 10 x 250 mm) and was operated using a linear gradient program of 90% A and 10% B for 10 minutes, then from 90% A and 10% B to 60% A and 40% B over 40 minutes (A = 0.1% TFA, B = acetonitrile) with detection at 220 nm and a flow rate of 3.5 ml/minute. Fractions were collected as set forth below.

**TABLE 5**

| | |
|---|---|
| Fraction 7-6.1 | 32.2 to 34.3 minutes. |
| Fraction 7-6.2 | 35.5 to 36.2 minutes. |

Each of the above fractions, from pooled runs, was concentrated by lyophilization.

### B) Subfractionation of Fraction 7-9

The Fraction 7-9 material from Step II-B, above, derived from ~300 µl of crude venom, was applied to a reversed phase HPLC column (Vydac®, C-18, 300 Å, 10 x 250 mm) and was operated using a linear gradient program of 90% A and 10% B for 10 minutes, then from 90% A and 10% B to 65% A and 35% B over 40 minutes (A = 0.1% TFA, B = acetonitrile) with detection at 220 nm and a flow rate of 3.5 ml/minute. Fraction 7-9.1 was collected and pooled from multiple runs with an elution time from 33 to 35 minutes.

### C) Subfractionation of Fraction 7-11

The Fraction 7-11 material from Step II-B, above, derived from ~600 µl of crude venom, was applied to a reversed phase HPLC column (Vydac®, C-18, 300 Å, 10 x 250 mm) and was operated using a linear gradient program of 90% A and 10% B for 10 minutes, then from 90% A and 10% B to 65% A and 35% B over 60 minutes (A = 0.1% TFA, B = acetonitrile) with detection at 220 nm and a flow rate of 3.5 ml/minute. Fraction 7-11.1 was collected and pooled from multiple runs with an elution time from 34.2 to 35.5 minutes.

### D) Subfractionation of Fraction 7-12

The Fraction 7-12 material from Step II-B, above, derived from ~600 µl of crude venom, was applied to a reversed phase HPLC column (Vydac®, C-18, 300 Å, 10 x 250 mm) and was operated using 90% A and 10% B for 10 minutes, then a linear gradient program from 90% A and 10% B to 65% A and 35% B over 60 minutes (A = 0.1% TFA, B = acetonitrile) with detection at 220 nm and a flow rate of 3.5 ml/minute. Fractions were collected as set forth below.

**TABLE 6**

| | |
|---|---|
| Fraction 7-12.1 | 28.0 to 29.1 minutes. |
| Fraction 7-12.2 | 38.4 to 39.6 minutes. |

### E) Subfractionation of Fraction 7-13

The Fraction 7-13 material from Step Il-B, above, derived from ~200 µl of crude venom, was applied to a reversed phase HPLC column (Vydac®, C-18, 300 Å, 10 x 250 mm) and was operated using 90% A and 10% B for 10 minutes, then a linear gradient program from 90% A and 10% B to 60% A and 40% B over 40 minutes (A = 0.1% TFA, B = acetonitrile) with detection at 220 nm and a flow rate of 3.5 ml/minute. Fractions were collected as set forth below.

**TABLE 7**

| | |
|---|---|
| Fraction 7-13.1 | 33.0 to 34.5 minutes. |
| Fraction 7-13.2 | 35.1 to 36.5 minutes. |

Each of the above fractions, from pooled runs, was concentrated by lyophilization.

### F) Subfractionation of Fraction 7-15

The Fraction 7-15 material from Step II-B, above, derived from ~200 µl of crude venom, was applied to a reversed phase HPLC column (Vydac®, C-18, 300 Å, 10 x 250 mm) and was operated using 90% A and 10% B for 10 minutes, then a linear gradient program from 90% A and 10% B to 60% A and 40% B over 40 minutes (A = 0.1% TFA, B = acetonitrile) with detection at 220 nm and a flow rate of 3.5 ml/minute. Fractions were collected as set forth below.

**TABLE 8**

| | |
|---|---|
| Fraction 7-15.1 | 33.1 to 33.6 minutes. |
| Fraction 7-15.2 | 34.5 to 36.2 minutes. |
| Fraction 7-15.3 | 37.7 to 39.1 minutes. |

Each of the above fractions, from pooled runs, was concentrated by lyophilization.

### G) Subfractionation of Fraction 7-16

The Fraction 7-16 material from Step II-B, above, derived from ~600 µl of crude venom, was applied to a reversed phase HPLC column (Vydac®, C-18, 300 Å, 10 x 250 mm) and was operated using 90% A and 10% B for 10 minutes, then a linear gradient program from 90% A and 10% B to 65% A and 35% B over 60 minutes (A = 0.1% TFA, B = acetonitrile) with detection at 220 nm and a flow rate of 3.5 ml/minute. Fraction 7-16.1 was collected and pooled from multiple runs with an elution time from 31.8 to 33.0 minutes.

### H) Subfractionation of Fraction 7-17

The Fraction 7-17 material from Step II-B, above, derived from ~25 µl of crude venom, was applied to a reversed phase HPLC column (Vydac®, C-18, 300 Å, 10 x 250 mm) and was operated using 90% A and 10% B for 10 minutes, then a linear gradient program from 90% A and 10% B to 60% A and 40% B over 40 minutes (A = 0.1% TFA, B = acetonitrile) with detection at 220 nm and a flow rate of 3.5 ml/minute. Fractions were collected as set forth below.

**TABLE 9**

| | |
|---|---|
| Fraction 7-17.1 | 15.6 to 18.4 minutes. |
| Fraction 7-17.2 | 18.5 to 19.4 minutes. |
| Fraction 7-17.3 | 27.1 to 28.6 minutes. |
| Fraction 7-17.4 | 28.6 to 30.1 minutes. |
| Fraction 7-17.5 | 30.1 to 31.9 minutes. |
| Fraction 7-17.6 | 31.9 to 33.5 minutes. |

Each of the above fractions, from pooled runs, was concentrated by lyophilization.

### Example 1 Aphonopelma peptide 6-6

The structure of peptide 6-6, prepared in Step Il-A, above, was determined and verified by the following methods. PTC amino acid analysis was carried out on 1-10 nmols in triplicate using the Waters Pico-Tag system. N-terminal sequencing was carried out on a pulse-liquid sequenator (ABI) on both native and reduced/pyridylethylated peptide. Mass spectral analysis was obtained from a SCI-EX API III ion spray mass spectrometer.

The data taken together affirm the structure of peptide 6-6 as shown below.
SEQ ID NO:1, 39 residues, 6 cysteines, 3 disulfide bonds.
Calculated mass = 4382.3.
Observed mass = 4382.16 ± 0.54 (ion spray m.s.).

### Example 2 Aphonopelma peptide 6-8

The structure of peptide 6-8, prepared in Step Il-A, above, was determined and verified by the following methods. PTC amino acid analysis was carried out on 1-10 nmols in triplicate using the Waters Pico-Tag system. N-terminal sequencing was carried out on a pulse-liquid sequenator (ABI) on both native and reduced/pyridylethylated peptide. Mass spectral analysis was obtained from a SCI-EX API III ion spray mass spectrometer.

The data taken together affirm the structure of peptide 6-8 as shown below.
SEQ ID NO:2, 39 residues, 6 cysteines, 3 disulfide bonds.
Calculated mass = 4369.2.
Observed mass = 4368.26 ± 0.27 (ion spray m.s.).

### Example 3 APhonopelma peptide 7-6.1

The structure of peptide 7-6.1, prepared in Step III-A, above, was determined and verified by the following methods. PTC amino acid analysis was carried out on 1-10 nmols in triplicate using the Waters Pico-Tag system. N-terminal sequencing was carried out on a pulse-liquid sequenator (ABI) on both native and reduced/pyridylethylated peptide. Mass spectral analysis was obtained from a SCI-EX API III ion spray mass spectrometer.

The data taken together affirm the structure of peptide 7-6.1 as shown below.
SEQ ID NO:3, 33 residues, 6 cysteines, 3 disulfide bonds.
Calculated mass = 3786.2.
Observed mass = 3784.54 (ion spray m.s.).

### Example 4 Aphonopelma peptide 7-13.1

The structure of peptide 7-13.1, prepared in Step III-E, above, was determined and verified by the following methods. PTC amino acid analysis was carried out on 1-10 nmols in triplicate using the Waters Pico-Tag system. N-terminal sequencing was carried out on a pulse-liquid sequenator (ABI) on both native and reduced/pyridylethylated peptide. Mass spectral analysis was obtained from a SCI-EX API III ion spray mass spectrometer.

The data taken together affirm the structure of peptide 7-13.1 as shown below.
SEQ ID NO:4, 34 residues, 6 cysteines, 3 disulfide bonds.
Calculated mass = 3814.32.
Observed mass = 3813.67 ± 0.27 (ion spray m.s.).

### Example 5 Aphonopelma peptide 7-13.2

The structure of peptide 7-13.2, prepared in Step III-E, above, was determined and verified by the following methods. PTC amino acid analysis was carried out on 1-10 nmols in triplicate using the Waters Pico-Tag system. N-terminal sequencing was carried out on a pulse-liquid sequenator (ABI) on both native and reduced/pyridylethylated peptide. Mass spectral analysis was obtained from a SCI-EX API III ion spray mass spectrometer.

The data taken together affirm the structure of peptide 7-13.2 as shown below.
SEQ ID NO:5, 42 residues, 6 cysteines, 3 disulfide bonds.
Calculated mass = 4844.45.
Observed mass = 4844.66 (ion spray m.s.).

### Example 6 Aphonopelma peptide 7-15.2

The structure of peptide 7-15.2, prepared in Step Ill-F, above, was determined and verified by the following methods. PTC amino acid analysis was carried out on 1-10 nmols in triplicate using the Waters Pico-Tag system. N-terminal sequencing was carried out on a pulse-liquid sequenator (ABI) on both native and reduced/pyridylethylated peptide. Mass spectral analysis was obtained from a SCI-EX API III ion spray mass spectrometer.

The data taken together affirm the structure of peptide 7-15.2 as shown below.
SEQ ID NO:6, 39 residues, 6 cysteines, 3 disulfide bonds.
Calculated mass = 4342.19.
Observed mass = 4341.84 ± 0.33 (ion spray m.s.).

### Example 7 Aphonopelma peptide 7-17.3

The structure of peptide 7-17.3, prepared in Step Ill-H, above, was determined and verified by the following methods. PTC amino acid analysis was carried out on 1-10 nmols in triplicate using the Waters Pico-Tag system. N-terminal sequencing was carried out on a pulse-liquid sequenator (ABI) on both native and reduced/pyridylethylated peptide. Mass spectral analysis was obtained from a SCI-EX API III ion spray mass spectrometer.

The data taken together affirm the structure of peptide 7-17.3 as shown below.
SEQ ID NO:7.
Observed mass = 4358.23 ± 0.47 (ion spray m.s.).

## Claims

1. A substantially pure polypeptide having the amino acid sequence, SEQ ID NO:1, SEQ ID NO:2, SEQ ID NO:3, SEQ ID NO:4, SEQ ID NO:5, SEQ ID NO:6, SEQ NO ID:7 or a pharmaceutically acceptable salt thereof.

2. A polypeptide as defined in claim 1, or a pharmaceutically acceptable salt thereof, for use as a pharmaceutical.

3. A pharmaceutical formulation comprising a polypeptide as defined in claim 1, or a pharmaceutically acceptable salt thereof, in admixture with a pharmaceutically acceptable adjuvant, diluent or carrier.

4. The use of a polypeptide as defined in claim 1, or a pharmaceutically acceptable salt thereof, in the manufacture of a medicament for the treatment of a calcium channel mediated disease.

5. The use as claimed in claim 4, wherein the disease is angina, hypertension, cardiomyopathies, supraventricular arrhythmias, aesophogeal achalasia, premature labour or Raynaud's disease.

## Patentansprüche

1. Im wesentlichen reines Polypeptid mit der Aminosäuresequenz SEQ ID Nr. 1, SEQ ID Nr. 2, SEQ ID Nr. 3, SEQ ID Nr. 4, SEQ ID Nr. 5, SEQ ID Nr. 6 oder SEQ ID Nr. 7 oder ein pharmazeutisch annehmbares Salz davon.

2. Polypeptid nach Anspruch 1 oder ein pharmazeutisch annehmbares Salz davon zur Verwendung als pharmazeutisches Mittel.

3. Pharmazeutisches Präparat umfassend ein Polypeptid, wie in Anspruch 1 definiert, oder ein pharmazeutisch annehmbares Salz davon in Mischung mit einem pharmazeutisch annehmbaren Hilfsstoff, Verdünnungsmittel oder Träger.

4. Verwendung eines Polypeptids nach Anspruch 1 oder eines pharmazeutisch annehmbaren Salzes davon zur Herstellung eines Arzneimittels zur Behandlung einer durch Calciumkanäle vermittelten Krankheit.

5. Verwendung nach Anspruch 4, worin die Krankheit Angina pectoris, Bluthochdruck, Kardiomyopathie, supraventrikuläre Arrhythmie, Aperistalsis oesophagi, vorzeitige Wehen oder Raynaud-Krankheit ist.

## Revendications

1. Polypeptide pratiquement pur, ayant la séquence d'amino-acides SEQ ID N° 1, SEQ ID N°2 ; SEQ ID N° 3, SEQ ID N° 4, SEQ ID N° 5, SEQ ID N° 6 ou SEQ ID N° 7, ou bien un de ses sels pharmaceutiquement acceptables.

2. Polypeptide répondant à la définition suivant la revendication 1 ou un de ses sels pharmaceutiquement acceptables, destiné à être utilisé comme agent pharmaceutique.

3. Formulation pharmaceutique comprenant un polypeptide répondant à la définition suivant la revendication 1 ou un de ses sels pharmaceutiquement acceptables, en mélange avec un adjuvant, diluant ou support pharmaceutiquement acceptable.

4. Utilisation d'un polypeptide répondant à la définition suivant la revendication 1 ou un de ses sels pharmaceutiquement acceptables, dans la production d'un médicament destiné au traitement d'une maladie à médiation par le canal calcium.

5. Utilisation suivant la revendication 4, dans laquelle la maladie consiste en angine de poitrine, hypertension, myocardiopathies, arythmies supraventriculaires, achalasie oesophagienne, travail prématuré ou maladie de Raynaud.
